# EUROPÄISCHE PATENTSCHRIFT

(11) **EP 2 827 144 B1**
(45) Veröffentlichungstag und Bekanntmachung des Hinweises auf die Patenterteilung: **31.01.2018**
(21) Anmeldenummer: 13177331.9
(22) Anmeldetag: 19.07.2013
(51) Int. Cl.: G01N 33/00

(54) **Sensorkopf für ein Gasmessgerät**
Sensor head for a gas measurement device
Tête de capteur pour un appareil de mesure de gaz

(43) Veröffentlichungstag der Anmeldung: 21.01.2015
(73) Patentinhaber: Dräger Safety AG & Co. KGaA, 23560 Lübeck (DE)
(72) Erfinder: Sürig, Andreas, 23628 Krummesse (DE)
(74) Vertreter: Uexküll & Stolberg

(56) Entgegenhaltungen:
- DE-C1- 19 503 802
- GB-A- 2 290 871
- US-A1- 2009 161 824
- US-A1- 2011 174 052
- US-A1- 2013 091 924

## Beschreibung

Die vorliegende Erfindung betrifft einen Sensorkopf für ein Gasmessgerät mit einem Gehäuse, das ein Innenvolumen umschließt, mit einer über eine Sensoröffnung im Gehäuse in Gasverbindung zu dem Innenvolumen stehenden, an dem Gehäuse vorgesehenen Sensorvorrichtung und mit einer an dem Gehäuse vorgesehenen gasdurchlässigen Membran, die vorgesehen ist, um von zur Sensorvorrichtung strömendem Gas durchströmt zu werden, mit einer in dem Gehäuse vorgesehenen Membranöffnung, die eine Gasverbindung zwischen dem Innenvolumen und der den Sensorkopf umgebenden Atmosphäre herstellt, wobei die gasdurchlässige Membran derart vor der Membranöffnung angeordnet ist, dass sie die Membranöffnung vollständig bedeckt.

Derartige Sensorköpfe für Gasmessgeräte, insbesondere tragbare Gasmessgeräte, können entweder im Diffusionsbetrieb oder im Pumpbetrieb eingesetzt werden. Im Diffusionsbetrieb strömt das nachzuweisende Gasgemisch üblicherweise durch wasserundurchlässige gasdurchlässige Membranen zu den Sensoren. Im Pumpbetrieb hingegen wird das Gas mittels Pumpen z.B. aus Tankanlagen angesaugt und über Schläuche zu den Sensoren gefördert.

Aus dem Stand der Technik, wie insbesondere der DE 76 18 326 U1, von der die vorliegende Erfindung ausgeht, sind ferner Sensorköpfe bekannt, die sowohl im Diffusionsbetrieb als auch im Pumpbetrieb verwendet werden können. Dabei strömt das nachzuweisende Gas im Pumpbetrieb durch eine Gaszuführung in ein Innenvolumen und von dort durch eine gasdurchlässige Membran zu Sensoren, während das Gas im Diffusionsbetrieb aus der den Sensorkopf umgebenden Atmosphäre unmittelbar durch die Membran zu den Sensoren strömt. Nachteilhaft bei einem solchen Sensorkopf ist jedoch, dass nicht in einfacher Weise zwischen den beiden Betriebszuständen umgeschaltet werden kann. Insbesondere kann im Pumpbetrieb ein Einströmen von Gas aus der umgebenden Atmosphäre in das Innenvolumen durch die Öffnung nicht vollkommen verhindert werden, wodurch wiederum Messergebnisse verfälscht werden können. Außerdem ist beim "Umschalten" zwischen den beiden Betriebsarten ein Umbau erforderlich, da das das Innenvolumen bildende Gehäuseteil entfernt werden muss.

Aus diesem Grunde sind im Stand der Technik ferner Sensorköpfe mit verschiedenartigen mechanischen Umschaltvorrichtungen vorgesehen, die z.B. als Zubehörteil, i.d.R. als Abdeckkappe, oder als in das Gehäuse integrierte mechanische Verschlusselemente ausgebildet sind. Derartige mechanische Umschaltvorrichtungen sind jedoch umständlich in der Handhabung, wartungsintensiv und unzuverlässig.

Aus der GB 2 290 871 A ist ein Gasmessgerät bekannt, bei dem das Messgas zunächst mit Hilfe einer Pumpe in eine erste Messkammer und dann durch einen Abscheider gezogen wird, wobei das Messgas schließlich in eine zweite Messkammer gefördert wird. Beide Messkammern sind durch eine Membran voneinander getrennt, und die zweite Messkammer ist mit der eigentlichen Sensorvorrichtung verbunden.

Die US 2009/0161824 A1 beschreibt lediglich, dass das Innenvolumen eines Sensorkopfes, das mit der Sensorvorrichtung, die hier als Detektor bezeichnet ist, direkt in Verbindung steht, durch eine Membran oder ein Filterelement verschlossen ist.

Schließlich ist aus der US 2011/0174052 lediglich bekannt, das Innenvolumen eines Sensorkopfes, in dem die eigentliche Sensorvorrichtung angeordnet ist, vollständig durch eine Membran und einen Filter abzuschließen.

Daher ist es die Aufgabe der vorliegenden Erfindung, einen Sensorkopf für ein Gasmessgerät bereitzustellen, der in möglichst einfacher und zuverlässiger Weise ein kontrolliertes Umschalten zwischen Pump- und Diffusionsbetrieb ermöglicht.

Diese Aufgabe wird durch die Merkmale des Anspruchs 1 gelöst.

In dem Gehäuse ist eine Membranöffnung vorgesehen, die eine Gasverbindung zwischen dem Innenvolumen und der den Sensorkopf umgebenden Atmosphäre herstellt, und dass die gasdurchlässige Membran derart vor der Membranöffnung angeordnet ist, dass sie die Membranöffnung vollständig bedeckt. Die Membran wird also nicht wie im Stand der Technik vor der Sensoröffnung, sondern vor der Membranöffnung angeordnet. Dabei wird der Querschnitt der Membranöffnung vorzugsweise so klein wie messtechnisch vertretbar ausgestaltet.

Auf diese Weise ist das Innenvolumen durch die Membran von der das Gehäuse umgebenden Atmosphäre getrennt. Das Gasgemisch kann zwar im Diffusionsbetrieb, d.h. wenn die Gaszuführung für das Einströmen von Gas in das Innenvolumen gesperrt ist, durch die gasdurchlässige, insbesondere wasserundurchlässige, Membran in das Innenvolumen und von dort zu der Sensorvorrichtung strömen. Im Pumpbetrieb jedoch, d.h. wenn Gas durch die Gaszuführung in das Innenvolumen gefördert wird, entsteht aufgrund der vor der Membranöffnung vorgesehenen gasdurchlässigen Membran in dem Innenvolumen ein leichter Überdruck gegenüber der den Sensorkopf umgebenden Atmosphäre, vorzugsweise von 1 bis 5 mbar über diesem Atmosphärendruck, sodass ein Einströmen bzw. Diffundieren von Gas geringeren Drucks aus der das Gehäuse des Sensorkopfes umgebenden Atmosphäre durch die in der Membranöffnung vorgesehenen Membran in das Innenvolumen aufgrund des Druckunterschieds zu dem Pumpgas im Innenvolumen verhindert wird. Somit ist im Pumpbetrieb in dem Innenvolumen nur Pumpgas enthalten, und Gas aus der umgebenden Atmosphäre kann das Messergebnis nicht verfälschen. Im Diffusionsbetrieb hingegen, wenn die Gaszuführung abgeschaltet ist, diffundiert das Gas der umgebenden Atmosphäre durch die Membran in das Innenvolumen und von dort zur Sensorvorrichtung.

Mit einem Sensorkopf solcher Gestalt kann daher in sehr einfacher und schneller Weise, durch ein bloßes An- bzw. Abschalten der Gasförderung durch die Gaszuführung in das Innenvolumen, kontrolliert zwischen Pumpbetrieb und Diffusionsbetrieb umgeschaltet werden. Es bedarf keines mechanischen Aufbaus, der für ein solches Umschalten bewegliche Teile erfordert. Der erfindungsgemäße Sensorkopf arbeitet ferner sehr zuverlässig und wartungsarm.

Erfindungsgemäß hat das Innenvolumen die Form einer sich parallel zur Ebene der durchströmten Querschnittsfläche der Sensoröffnung erstreckenden Scheibe, vorzugsweise einer Kreisscheibe. Mit einer solchen Form des Innenvolumens hat es sich gezeigt, dass die Sensorvorrichtung sowohl im Pumpbetrieb als auch im Diffusionsbetrieb schnell und zuverlässig auf Konzentrationsänderungen im jeweiligen Messgas anspricht.

In einer weiteren bevorzugten Ausführungsform ist die Abmessung des Innenvolumens in Richtung einer Flächennormalen auf der durchströmten Querschnittsfläche der Sensoröffnung wenigstens um einen Faktor, der größer als 5 ist, kleiner als die Abmessungen des Innenvolumens parallel zu dieser Querschnittsfläche. Derartige Abmessungen des Innenvolumens führen im Pumpbetrieb zu einem besonders effektiven "Blockieren" der Membranöffnung bzw. der Membran für aus der das Gehäuse umgebenden Atmosphäre in das Innenvolumen einströmendes bzw. eindiffundierendes Gas, sodass das Messergebnis im Pumpbetrieb nicht verfälscht wird.

Erfindungsgemäß sind die Sensoröffnung und die Membranöffnung einander gegenüberliegend in dem Gehäuse vorgesehen und stehen durch das Innenvolumen, das als senkrecht zur Flächennormalen auf der durchströmten Querschnittsfläche der Sensoröffnung verlaufenden Scheibe ausgebildete ist, in Gasverbindung. Eine gegenüberliegende Anordnung von Membranöffnung und Sensoröffnung dient dazu, dass im Diffusionsbetrieb des Sensorkopfes das aus der das Gehäuse umgebenden Atmosphäre durch die Membran in das Innenvolumen diffundierende Gas möglichst direkt, d.h. ohne Umwege, durch die Sensoröffnung in die Sensorvorrichtung strömen kann. Auf diese Weise wird eine möglichst hohe Messgenauigkeit und Messgeschwindigkeit des Sensorkopfes im Diffusionsbetrieb erreicht.
Erfindungsgemäß weist das Gehäuse einen gegenüber der zentralen Flächennormalen auf der durchströmten Querschnittsfläche der Sensoröffnung konzentrisch um das Innenvolumen ausgebildeten Ringkanal auf, wobei der Ringkanal in Gasverbindung steht zu dem Innenvolumen und wobei der Ringkanal in Gasverbindung mit der Gaszuführung steht, und der Ringkanal mit dem Innenvolumen über eine Vielzahl von zu der Flächennormalen auf der durchströmten Querschnittsfläche der Sensoröffnung radial verlaufenden Verbindungskanälen verbunden ist. Insbesondere ist es bevorzugt, wenn die Verbindungskanäle sich gleichmäßig voneinander beabstandet, z.B. vier Verbindungskanäle mit einem Winkel von 90° voneinander beabstandet, von dem Ringkanal radial zur Flächennormalen auf der durchströmten Querschnittsfläche der Sensoröffnung zum Innenvolumen erstrecken. Die zentrale Flächennormale erstreckt sich dabei durch die Mitte der Sensoröffnung.

Durch einen solchen Ringkanal, durch den im Pumpbetrieb das über die Gaszuführung in das Innenvolumen geförderte Gas strömt, wird das Innenvolumen effektiv "gespült", sodass Änderungen in der Zusammensetzung des Pumpgases schnell von der Sensorvorrichtung erfasst werden.

Vorzugsweise ist auf der zum Innenvolumen weisenden Seite der gasdurchlässigen Membran eine gasundurchlässige Folie oder Abdeckung angebracht, die eine Öffnung definierter Abmessung aufweist. Auf diese Weise wird eine definierte Querschnittsfläche vorbestimmter Größe der vor der Membranöffnung vorgesehenen Membran festgelegt, durch die das Gas aus der das Gehäuse umgebenden Atmosphäre im Diffusionsbetrieb des Sensorkopfes in das Innenvolumen diffundieren kann, und es wird ein Hindurchtreten einer definierten Menge an Gas zugelassen. Die Abmessungen dieser Öffnung in der Folie bzw. Abdeckung sind dabei vorzugsweise so klein wie messtechnisch gerade noch möglich zuwählen.

Die Gaszuführung ist in einer weiteren bevorzugten Ausführungsform mit einer Gaspumpvorrichtung zur Förderung von Gas in das Innenvolumen verbunden. Eine solche Pumpvorrichtung kann z.B. eine Membranpumpe sein. Die Pumpvorrichtung ist dabei derart auszulegen, dass sie einerseits möglichst klein, d.h. möglichst platzsparend, ist und auf der anderen Seite so viel Gas wie messtechnisch erforderlich aus einem Behälter, d.h. auch aus einem größeren Behälter wie z.B. einem Gastank, in das Innenvolumen des Sensorkopfes gefördert werden kann.

In noch einer weiteren bevorzugten Ausführungsform ist auf der von dem Innenvolumen abgewandten Seite der gasdurchlässigen Membran ein die Membran auf dieser Seite vollständig umgebender Schutzgaskanal vorgesehen, wobei der Schutzgaskanal über eine Kanalöffnung mit der umgebenden Atmosphäre in Verbindung steht, wobei der Schutzgaskanal eine Schutzgaszufuhröffnung aufweist und wobei die Schutzgaszuführöffnung mit der Gaszuführung verbunden ist.

Auf diese Weise wird im Pumpbetrieb das nachzuweisende Gas von der Gaszuführung nicht nur in das Innenvolumen, sondern auch über die Schutzgaszufuhröffnung in den Schutzgaskanal gefördert, von wo es durch die Kanalöffnung in die den Sensorkopf umgebende Atmosphäre ausströmen kann. Gas aus der den Sensorkopf umgebenden Atmosphäre kann dabei gar nicht erst in den Schutzgaskanal einströmen, da es von dem aus der Schutzgaszufuhröffnung einströmenden Gas verdrängt wird, und somit auch nicht zu der Membran gelangen. Im Diffusionsbetrieb hingegen, wenn kein Gas aus der Schutzgaszufuhröffnung in den Schutzgaskanal gefördert wird, kann das Gas aus der den Sensorkopf umgebenden Atmosphäre in den Schutzgaskanal einströmen und von dort zu der Membranöffnung gelangen, durch die gasdurchlässige Membran in das Innenvolumen strömen und von dort durch die Sensoröffnung in die Sensorvorrichtung gelangen.

Ein solcher der Membran vorgeschalteter Schutzgaskanal dient allgemein dem Schutz der Membran, insbesondere vor externen Gasströmungen, wie Winden, die gegen die Membran drücken, d.h. auf der vom Innenvolumen abgewandten Seite der Membran vorübergehend einen erhöhten Druck aufbauen, und auf diese Weise im Pumpbetrieb ein Diffundieren von Gas durch die Membran in das Innenvolumen verursachen könnten, wodurch wiederum das Messergebnis verfälscht werden könnte.

Dabei ist es besonders bevorzugt, wenn die Kanalöffnung der Membranöffnung gegenüber liegt. Eine gegenüberliegende Anordnung von Kanalöffnung und Membranöffnung dient dazu, dass im Diffusionsbetrieb Gas aus der den Sensorkopf umgebenden Atmosphäre möglichst direkt und geradlinig von der Kanalöffnung durch den Schutzgaskanal zu der Membranöffnung strömen und von dort durch die Membran in das Innenvolumen diffundieren kann.

Besonders bevorzugt ist es auch, wenn die Kanalöffnung durch eine gasdurchlässige Schutzmembran abgedeckt ist. Eine solche vor der Kanalöffnung vorgesehene Schutzmembran bietet einen weiteren Schutz der vor der Membranöffnung vorgesehenen Membran vor Windeinflüssen. Gas aus der den Sensorkopf umgebenden Atmosphäre muss auf diese Weise zunächst durch die Schutzmembran diffundieren, danach durch den Schutzgaskanal zu der Membranöffnung strömen, dann wiederum durch die Membran diffundieren, um schließlich durch das Innenvolumen zur Sensoröffnung und in die Sensorvorrichtung strömen zu können. Eine Verfälschung von Messergebnissen während des Pumpbetriebs durch aufgrund von Wind durch die Membran gedrückten bzw. diffundierenden Gases aus der den Sensorkopf umgebenden Atmosphäre kann somit weitgehend verhindert werden.

Ebenso ist es dabei besonders bevorzugt, wenn auf der von dem Schutzgaskanal abgewandten Seite der Kanalöffnung eine Schutzblende vorgesehen ist, die eine Vielzahl von Durchlassöffnungen zum Durchlass von Gas aus dem Schutzgaskanal zur die Schutzblende umgebenden Atmosphäre aufweist. Eine solche Schutzblende stellt eine weitere Maßnahme zum Schutz der Membran vor Windeinflüssen dar und begrenzt den Schutzgaskanal auf der Seite gegenüber der Membranöffnung. Die Schutzblende ist vorzugsweise als starre Struktur ausgebildet und die Durchlassöffnungen sind derart bemessen, dass im Pumpbetrieb das durch die Schutzgaszufuhröffnung in den Schutzgaskanal einströmende Gas, ohne sich zu stauen durch diese entweichen kann und dass im Diffusionsbetrieb messtechnisch ausreichend Gas aus der den Sensorkopf umgebenden Atmosphäre in den Schutzgaskanal und dann weiter in das Innenvolumen und in die Sensorvorrichtung strömen kann. Eine solche Schutzblende kann auch zusätzlich zu der Schutzmembran vor der Kanalöffnung vorgesehen sein.

Alternativ ist es auch bevorzugt, wenn auf der von dem Schutzgaskanal abgewandten Seite der Kanalöffnung eine Windschutzeinrichtung vorgesehen ist. Eine solche Windschutzeinrichtung kann zusätzlich zu einer vor der Kanalöffnung vorgesehenen Schutzmembran beabstandet zu dieser vorgesehen werden, um noch einen weitergehenden Schutz vor Windeinflüssen für die Membran und damit noch sicherere Messergebnisse zu erzielen. Die Windschutzeinrichtung kann z.B. in Form von mehreren parallel zueinander angeordneten flächigen Strukturen mit einer Vielzahl gegeneinander verschobener Durchlässe ausgebildet sein.

Die vorliegende Erfindung wird im Folgenden anhand einer vier verschiedene Ausführungsbeispiele darstellenden Zeichnung erläutert. Die Zeichnung zeigt in
- Fig. 1: eine auseinandergezogene Darstellung eines ersten Ausführungsbeispiels eines erfindungsgemäßen Sensorkopfs,
- Fig. 2: eine aufgeschnittene perspektivische Darstellung des Sensorkopfs aus Fig. 1,
- Fig. 3: eine auseinandergezogene Darstellung eines zweiten Ausführungsbeispiels eines erfindungsgemäßen Sensorkopfs,
- Fig. 4: eine aufgeschnittene perspektivische Darstellung des Sensorkopfs aus Fig. 3,
- Fig. 5: eine auseinandergezogene Darstellung eines dritten Ausführungsbeispiels eines erfindungsgemäßen Sensorkopfs,
- Fig. 6: eine aufgeschnittene perspektivische Darstellung des Sensorkopfs aus Fig. 5 und
- Fig. 7: eine Querschnittsansicht eines vierten Ausführungsbeispiels eines erfindungsgemäßen Sensorkopfes.

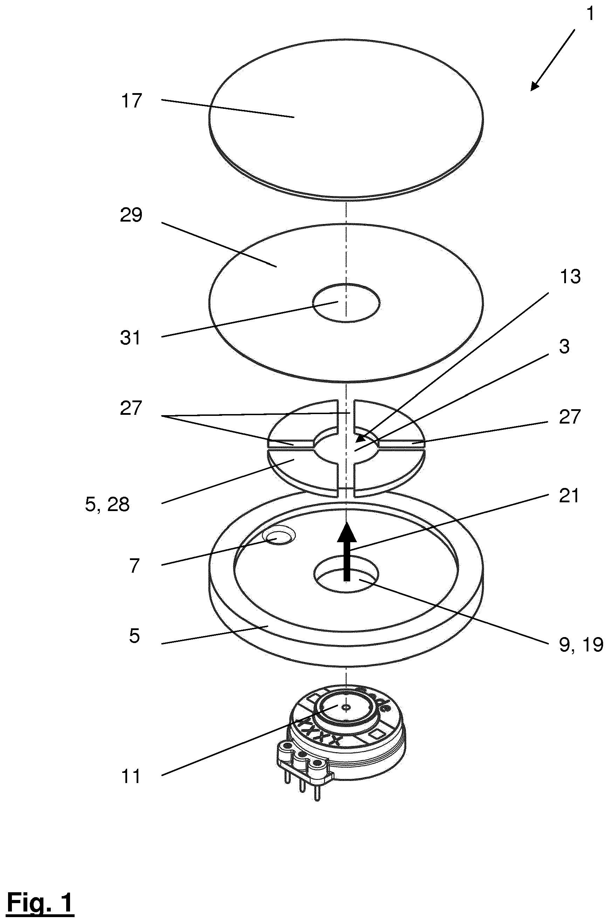

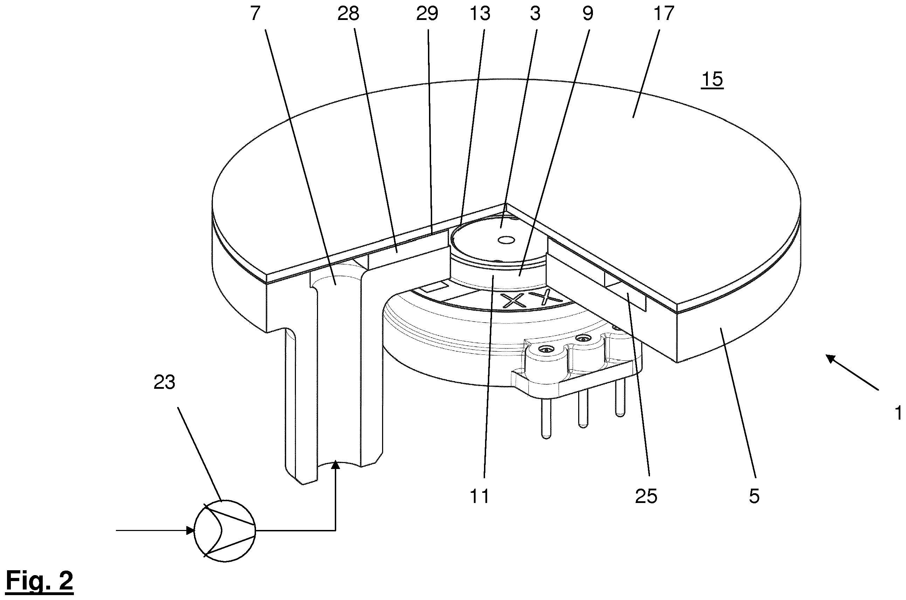

In Fig. 1 und 2 ist ein erstes Ausführungsbeispiel des erfindungsgemäßen Sensorkopfs 1 für ein Gasmessgerät dargestellt. Der Sensorkopf 1 umfasst ein ein Innenvolumen 3 umgebendes Gehäuse 5, eine in dem Gehäuse 5 ausgebildete Gaszuführung 7 zur Zufuhr von Gas in das Innenvolumen 3, eine in dem Gehäuse 5 ausgebildete Sensoröffnung 9, an der in Gasverbindung zu dem Innenvolumen 3 eine Sensorvorrichtung 11 vorgesehen ist, und eine gegenüber der Sensoröffnung 9 in dem Gehäuse 5 ausgebildete Membranöffnung 13, durch die das Innenvolumen mit der das Gehäuse 5 umgebenden Atmosphäre 15 verbunden ist und die von einer gasdurchlässigen und vorzugsweise wasserundurchlässigen Membran 17 vollständig bedeckt ist.

Das Innenvolumen 3 weist dabei die Form einer sich parallel zur Ebene der durchströmten Querschnittfläche 19 der Sensoröffnung 9 erstreckenden Scheibe, in dem vorliegenden Ausführungsbeispiel einer Kreisscheibe, auf. Die Abmessungen der Kreisscheibe in Richtung einer Flächennormalen 21 auf der durchströmten Querschnittsfläche 19 der Sensoröffnung 9 sind dabei wenigstens um einen Faktor 5, vorliegend um einen Faktor 10, kleiner als die Abmessungen des Innenvolumens 3 bzw. der Kreisscheibe parallel zu dieser Querschnittsfläche 19. Ferner ist eine Gaspumpvorrichtung 23 vorgesehen (siehe Fig. 2), die im Pumpbetrieb des Sensorkopfes 1 bzw. des Gasmessgerätes zur Förderung von Gas in das Innenvolumen 3 mit der Gaszuführung 7 verbunden ist.

Das in Fig. 1 und 2 dargestellte erste Ausführungsbeispiel umfasst ferner einen in dem Gehäuse 5 ausgebildeten gegenüber der zentralen Flächennormalen 21 auf der durchströmten Querschnittsfläche 19 der Sensoröffnung 9 konzentrisch um das Innenvolumen 3 ausgebildeten Ringkanal 25, der in Gasverbindung sowohl zu dem Innenvolumen 3 als auch zu der Gaszuführung 7 steht. Die zentrale Flächennormale 21 erstreckt sich dabei durch die Mitte der Sensoröffnung 9. Die Gaszuführung 7 mündet vorliegend direkt in dem Ringkanal 25, während das Innenvolumen 3 mit dem Ringkanal 25 über eine Vielzahl von Verbindungskanälen 27 verbunden ist, die sich von dem Ringkanal 25 in radialer Weise zu dem Innenvolumen 3 erstrecken, d.h. radial zu der Flächennormalen 21 auf der durchströmten Querschnittsfläche 19 der Sensoröffnung 9 verlaufen. In dem vorliegenden Ausführungsbeispiel sind, wie in Fig. 1 dargestellt, vier Verbindungskanäle 27 vorgesehen, die sich gleichmäßig um 90° voneinander beabstandet in einer gemeinsamen Ebene parallel zur durchströmten Querschnittsfläche 19 der Sensoröffnung 9 von dem Ringkanal 25 zu dem Innenvolumen 3 erstrecken. Um den Ringkanal 25 gegenüber dem Innenvolumen 3 abzugrenzen, ist eine ringförmige Zwischenwand 28 vorgesehen, in welcher die Verbindungskanäle 27 ausgebildet sind.

Um eine definierte Querschnittsfläche vorbestimmter Größe der vor der Membranöffnung 13 vorgesehenen Membran 17 festzulegen, durch die das Gas aus der das Gehäuse 5 umgebenden Atmosphäre 15 im Diffusionsbetrieb des Sensorkopfes 1 in das Innenvolumen 3 diffundieren kann, ist auf der zum Innenvolumen 3 weisenden Seite der gasdurchlässigen Membran 17 eine gasundurchlässige Folie 29 oder Abdeckung angebracht, die eine Öffnung 31 definierter Abmessung aufweist, um auf diese Weise ein Hindurchtreten einer definierten Menge an Gas zuzulassen. Die Abmessungen dieser Öffnung 31 in der Folie 29 bzw. Abdeckung sind dabei so klein wie messtechnisch gerade noch möglich.

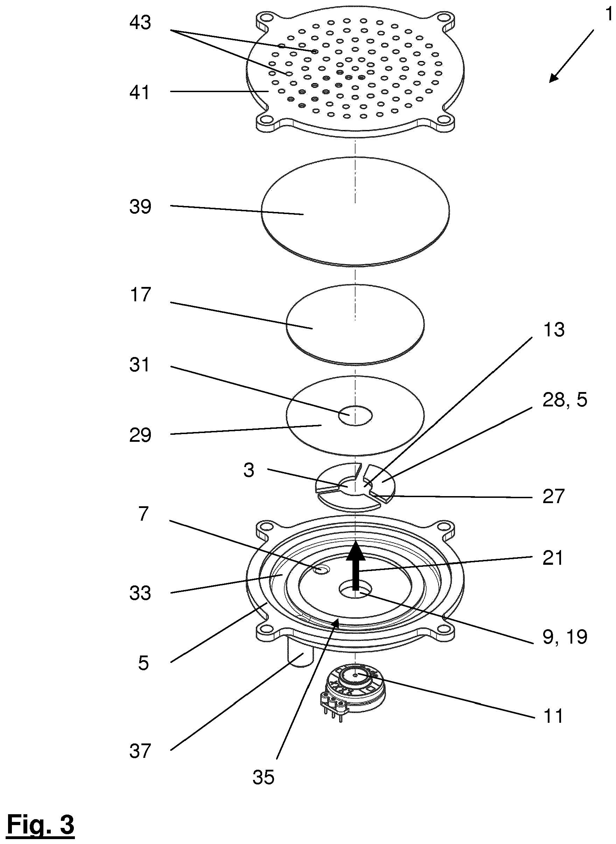

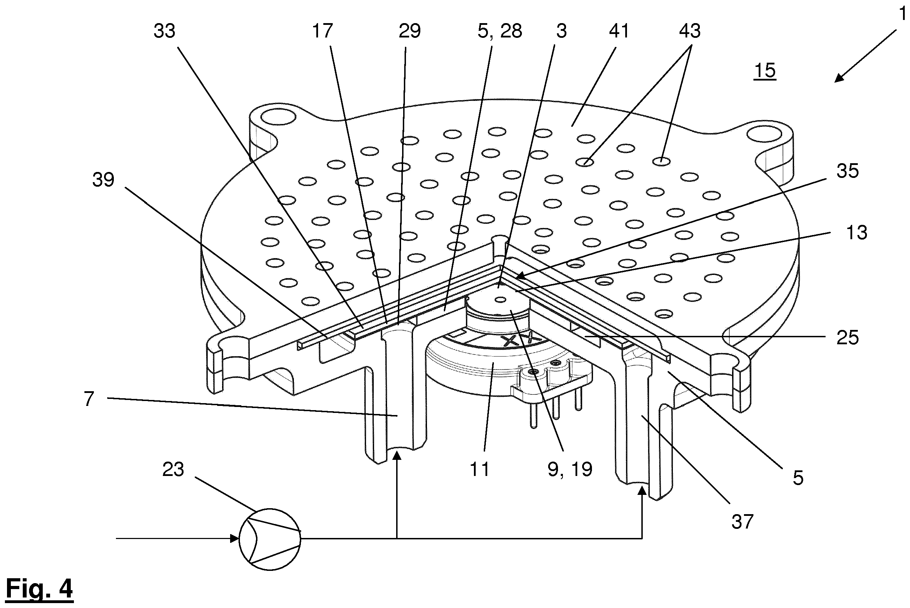

In Fig. 3 und 4 ist ein zweites Ausführungsbeispiel des erfindungsgemäßen Sensorkopfs 1 für ein Gasmessgerät dargestellt, das sich nur in wenigen Merkmalen von dem ersten Ausführungsbeispiel unterscheidet, sodass entsprechende Merkmale mit gleichen Bezugszeichen bezeichnet sind.

Das in Fig. 3 und 4 dargestellte zweite Ausführungsbeispiel umfasst über die Merkmale des ersten Ausführungsbeispiels hinaus auf der von dem Innenvolumen 3 abgewandten Seite der gasdurchlässigen Membran 17 einen die Membran 17 auf dieser Seite vollständig umgebenden Schutzgaskanal 33, der wiederum eine der Membranöffnung 13 gegenüberliegende und sich bis über den Ringkanal 25 hinaus erstreckende Kanalöffnung 35, über die der Schutzgaskanal 33 mit der den Sensorkopf 1 umgebenden Atmosphäre 15 verbunden ist, und eine Schutzgaszufuhröffnung 37 aufweist, die mit der Gaszuführung 7 verbunden ist. Die Kanalöffnung 35 ist ferner mittels einer gasdurchlässigen, wasserundurchlässigen Schutzmembran 39 abgedeckt, wodurch der Schutzgaskanal 33 zur den Sensorkopf 1 umgebenden Atmosphäre 15 abgegrenzt ist und lediglich eine Diffusion von Gas durch die Schutzmembran 39 möglich ist.

Darüber hinaus ist auf der von dem Schutzgaskanal 33 abgewandten Seite der Schutzmembran 39 eine starre Schutzblende 41 vorgesehen ist, die eine Vielzahl von Durchlassöffnungen 43 zum Durchlass von Gas aus dem Schutzgaskanal 33 zur die Schutzblende 41 bzw. den Sensorkopf 1 umgebenden Atmosphäre 15 aufweist.

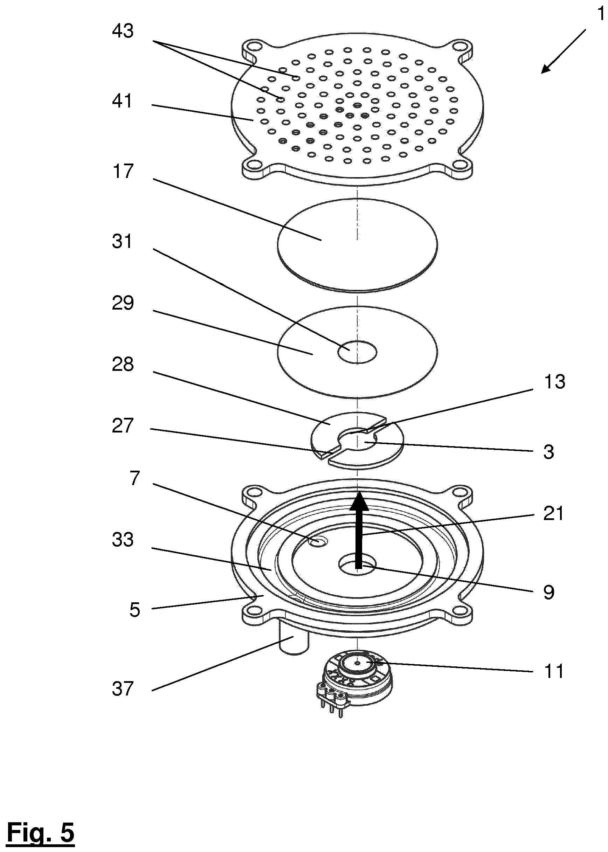

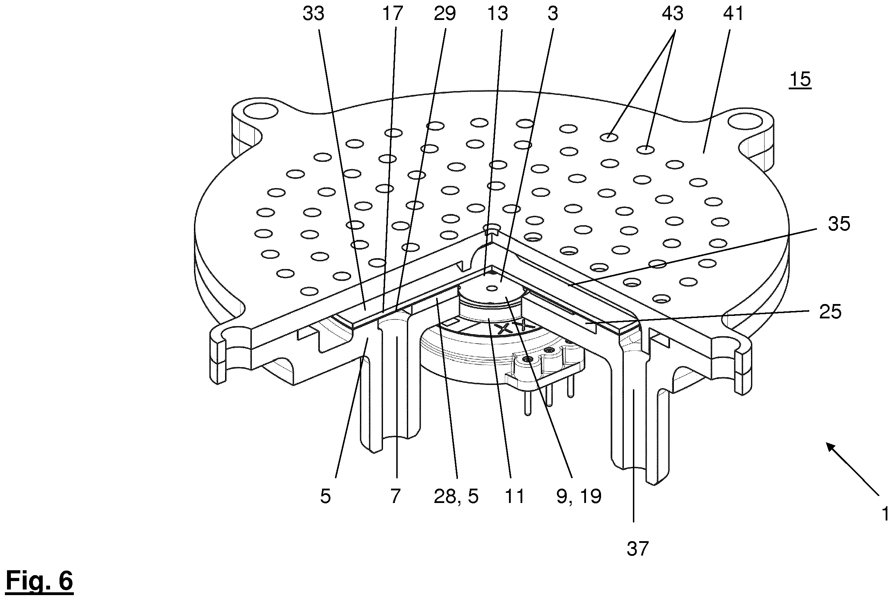

In Fig. 5 und 6 ist ein drittes Ausführungsbeispiel des erfindungsgemäßen Sensorkopfs 1 für ein Gasmessgerät dargestellt, das sich nur in wenigen Merkmalen von dem ersten und zweiten Ausführungsbeispiel unterscheidet, sodass entsprechende Merkmale mit gleichen Bezugszeichen bezeichnet sind.

Das in Fig. 5 und 6 dargestellte dritte Ausführungsbeispiel ist ähnlich dem zweiten Ausführungsbeispiel ausgebildet, abgesehen davon, dass zur Abdeckung der Kanalöffnung 35 auf der von dem Schutzgaskanal 33 abgewandten Seite der Kanalöffnung 35 keine Schutzmembran 39 sondern ausschließlich eine starre Schutzblende 41 vorgesehen ist, die eine Vielzahl von Durchlassöffnungen 43 zum Durchlass von Gas aus dem Schutzgaskanal 33 zur die Schutzblende 41 bzw. den Sensorkopf 1 umgebenden Atmosphäre 15 aufweist.

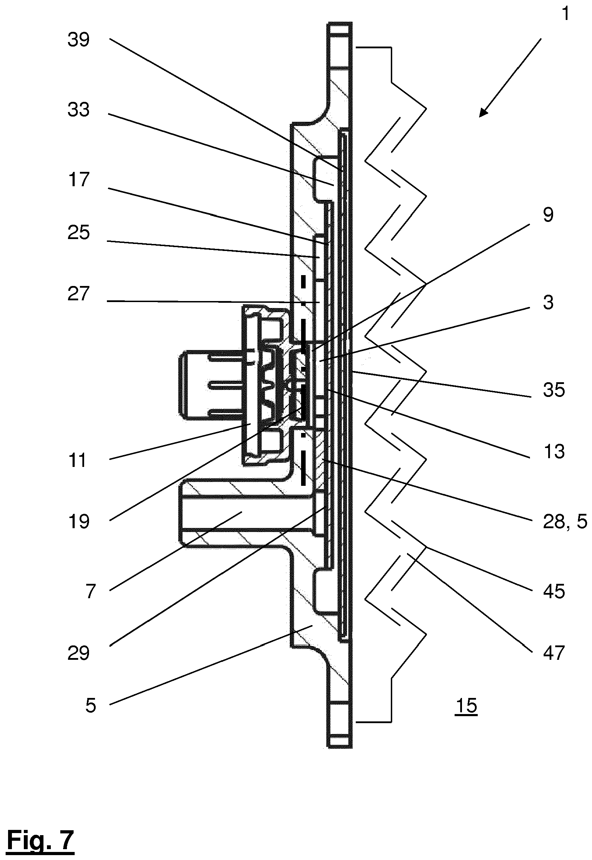

In Fig. 7 ist ein viertes Ausführungsbeispiel des erfindungsgemäßen Sensorkopfs 1 für ein Gasmessgerät dargestellt, das sich nur in wenigen Merkmalen von dem ersten, zweiten und dritten Ausführungsbeispiel unterscheidet, sodass entsprechende Merkmale mit gleichen Bezugszeichen bezeichnet sind.

Das in Fig. 7 dargestellte vierte Ausführungsbeispiel ist ähnlich dem zweiten Ausführungsbeispiel ausgebildet, abgesehen davon, dass auf der von dem Schutzgaskanal 33 abgewandten Seite der Schutzmembran 39 keine Schutzblende 41 sondern parallel beabstandet zu der Schutzmembran 39 eine Windschutzeinrichtung 45 vorgesehen ist, die mehrere - vorliegend zwei - parallel zueinander angeordnete flächige Strukturen mit zick-zackförmigem Querschnitt und einer Vielzahl gegeneinander verschobener, d.h. nicht miteinander fluchtender Durchlässe 47 aufweist.

Der Sensorkopf 1 für ein Gasmessgerät gemäß dem ersten Ausführungsbeispiel (siehe Fig. 1 und 2) kann wie folgt verwendet werden. Im Pumpbetrieb fördert die Gaspumpvorrichtung 23 das zu untersuchende Gas bzw. Gasgemisch z.B. aus einer Tankanlage über Rohre oder Schläuche durch die Gaszuführung 7 über den Ringkanal 25 und die Verbindungskanäle 27 in das Innenvolumen 3. Von dort kann das Gas dann durch die Sensoröffnung 9 in die Sensorvorrichtung 11 strömen, wo ein Nachweis der Gasart von Interesse stattfinden kann.

Im Diffusionsbetrieb hingegen ist die Gaspumpvorrichtung 23 abgeschaltet, wobei diese dann wie ein Ventil wirkt, sodass über die Gaszuführung 7 kein Zustrom von Gas erfolgt, und das nachzuweisende Gas bzw. Gasgemisch diffundiert aus der das Gehäuse 5 des Sensorkopfes 1 umgebenden Atmosphäre 15 durch die vor der Membranöffnung 13 vorgesehene gasdurchlässige Membran 17 und gelangt somit durch die Membranöffnung 13 in das Innenvolumen 3 und von dort durch die Sensoröffnung 9 in die Sensorvorrichtung 11, wo der Nachweis des Gases erfolgen kann.

Damit in kontrollierter und verlässlicher Weise zwischen Pumpbetrieb und Diffusionsbetrieb umgeschaltet werden kann, d.h. im Pumpbetrieb kein Gas aus der den Sensorkopf 1 umgebenden Atmosphäre 15 und im Diffusionsbetrieb kein Gas aus der an die Gaszuführung 7 angeschlossenen Leitung oder Tankanlage zur Sensorvorrichtung 11 gelangt und auf diese Weise das Messergebnis verfälschen könnte, kann im Diffusionsbetrieb auch ein an der Gaspumpvorrichtung 23 oder an der an die Gaszuführung 7 angeschlossenen Leitung vorgesehenes Ventil gesperrt werden. Im Pumpbetrieb hingegen wird außerdem ein Diffundieren von Gas aus der umgebenden Atmosphäre 15 durch die in der Membranöffnung 13 vorgesehenen Membran 17 dadurch verhindert, dass zum einen die Querschnittsfläche der Membran 17 bzw. der Öffnung 31 der gasundurchlässigen Folie 29 oder Abdeckung, durch die Gas aus der umgebenden Atmosphäre 15 in das Innenvolumen 3 diffundieren könnte, möglichst klein gehalten und zum anderen das Innenvolumen 3 in flacher scheibenartiger Form ausgebildet sind, wodurch sich im Innenvolumen 3 vor der Membran 17 ein gegenüber der den Sensorkopf 1 umgebenden Atmosphäre 15 leichter Überdruck von ca. 1 bis 5 mbar einstellt, was wiederum einem Diffundieren von Gas durch die Membran 17 in das Innenvolumen 3 entgegenwirkt.

Bei einem solchen Aufbau des Sensorkopfes 1, wo um das Innenvolumen 3 in dem Gehäuse 5 konzentrisch ein Ringkanal 25 gebildet ist, der mit dem Innenvolumen 3 über radial verlaufende Verbindungskanäle 27 verbunden ist und durch den das von der Gaspumpvorrichtung 23 über die Gaszuführung 7 in das Innenvolumen 3 geförderte Gas auf dem Weg in das Innenvolumen 3 strömen muss, wird das Innenvolumen 3 und die Sensorvorrichtung 11 effektiv mit neu zugeführtem Gas gespült, sodass Änderungen in der Zusammensetzung des Pumpgases zuverlässig erfasst werden.

Die Festlegung einer genau vorbestimmten möglichst kleinen Querschnittsfläche der Membran 17, durch die das Gas im Diffusionsbetrieb in das Innenvolumen 3 diffundieren kann, wird durch Aufbringen einer gasundurchlässigen Folie 29 oder Abdeckung an der zum Innenvolumen 3 gewandten Seite der Membran 17 erreicht.

Gemäß dem zweiten, dritten und vierten Ausführungsbeispiel können die Wirkungen des zuvor beschriebenen ersten Ausführungsbeispiels dahingehend verbessert werden, dass der Sensorkopf 1 weniger abhängig von äußeren Einflüssen, insbesondere Wind- bzw. Anströmungseinflüssen, wird und dass die Messgenauigkeit erhöht wird. Dafür sehen sowohl die zweite und dritte als auch die vierte Ausführungsform einen Schutzgaskanal 33 vor, welcher der Membran 17 vorgeschaltet ist, d.h. sich auf der vom Innenvolumen 3 abgewandten Seite der Membran 17 an dieser entlang erstreckt, durch den ein ebenfalls von der Gaspumpvorrichtung 23 gefördertes und von der Gaszuführung 7 abgezweigtes Gas an der vom Innenvolumen 3 abgewandten Seite der Membran 17 entlang strömt, und diese somit vor aus der den Sensorkopf 1 umgebenden Atmosphäre 15 anströmendem, auf die Membran 17 drückendem Wind schützt, der sonst eine Diffusion von Gas durch die Membran 17 in das Innenvolumen 3 auch während des Pumpbetriebs hervorrufen bzw. verstärken könnte.

Das zweite bis vierte Ausführungsbeispiel unterscheidet sich jedoch in dem Abschluss der Kanalöffnung 35 zur den Sensorkopf 1 umgebenden Atmosphäre 15. Das zweite Ausführungsbeispiel sieht vor der Kanalöffnung 35 eine Schutzmembran 39 vor, durch die aufgrund von Windeinflüssen in den Schutzgaskanal 33 eindringendes Gas auf dem Weg zur Sensorvorrichtung 11 zunächst diffundieren müsste, wodurch ein weiterer Windschutz zusammen mit einer noch höheren Messgenauigkeit erzielt wird. Obendrein ist in dem zweiten Ausführungsbeispiel noch eine mit Durchlassöffnungen 43 versehene starre Schutzblende 41 vorgesehen, die die zuvor beschriebene Windschutzwirkung noch verstärkt, da Wind aus der den Sensorkopf 1 umgebenden Atmosphäre 15 auf dem Weg zur Sensorvorrichtung 11 grundsätzlich nur durch die Durchlassöffnungen 43 in den Schutzgaskanal 33 strömen kann, die im Pumpbetrieb des Sensorkopfes 1 jedoch von Gas in umgekehrter Richtung, d.h. aus dem Schutzgaskanal 33 in die umgebende Atmosphäre 15 durchströmt werden, was ein unerwünschtes Eindringen von Gas in den Schutzgaskanal 33 aufgrund von Wind verhindert bzw. erschwert.

Wie in dem dritten Ausführungsbeispiel beschrieben, kann die Schutzblende 41 jedoch auch alleine, d.h. ohne eine Schutzmembran 39 zur Abdeckung der Kanalöffnung 35 verwendet werden. Dadurch kann der Aufbau des Sensorkopfes 1 etwas vereinfacht werden, ohne dass die Windschutzwirkungen in hohem Maße abnehmen.

Alternativ hierzu, wie in dem vierten Ausführungsbeispiel beschrieben, kann jedoch auch eine Schutzmembran 39 alleine ohne eine Schutzblende 41 eingesetzt werden. Ein zusätzlicher Windschutz kann dabei durch die in Fig. 7 dargestellte Windschutzeinrichtung 45 erreicht werden, welche die direkte Anströmung des Windes auf die Schutzmembran 39 abwehrt und nur an vereinzelten Stellen anströmendes Gas über Umwege durch die Durchlässe 47 zu der Schutzmembran 39 durchlässt.

### Bezugszeichenliste:

- 1: Sensorkopf
- 3: Innenvolumen
- 5: Gehäuse
- 7: Gaszuführung
- 9: Sensoröffnung
- 11: Sensorvorrichtung
- 13: Membranöffnung
- 15: Atmosphäre
- 17: Membran
- 19: durchströmte Querschnittsfläche der Sensoröffnung
- 21: Flächennormale auf der durchströmten Querschnittsfläche
- 23: Gaspumpvorrichtung
- 25: Ringkanal
- 27: Verbindungskanäle
- 28: Zwischenwand
- 29: Folie
- 31: Öffnung in der Folie
- 33: Schutzgaskanal
- 35: Kanalöffnung
- 37: Schutzgaszufuhröffnung
- 39: Schutzmembran
- 41: Schutzblende
- 43: Durchlassöffnungen in der Schutzblende
- 45: Windschutzeinrichtung
- 47: Durchlässe in der Windschutzeinrichtung

## Patentansprüche

1. Sensorkopf (1) für ein Gasmessgerät
mit einem Gehäuse (5), das ein Innenvolumen (3) umschließt,
mit einer über eine Sensoröffnung (9) im Gehäuse (5) in Gasverbindung zu dem Innenvolumen (3) stehenden, an dem Gehäuse (5) vorgesehenen Sensorvorrichtung (11) und
mit einer an dem Gehäuse (5) vorgesehenen gasdurchlässigen Membran (17), die vorgesehen ist, um von zur Sensorvorrichtung (11) strömendem Gas durchströmt zu werden,
mit einer in dem Gehäuse (5) vorgesehenen Membranöffnung (13), die eine Gasverbindung zwischen dem Innenvolumen (3) und der den Sensorkopf (1) umgebenden Atmosphäre (15) herstellt,
wobei die gasdurchlässige Membran (17) derart vor der Membranöffnung (13) angeordnet ist, dass sie die Membranöffnung (13) vollständig bedeckt,
**dadurch gekennzeichnet,**
**dass** der Sensorkopf eine in dem Gehäuse (5) vorgesehenen Gaszuführung (7) zur Zufuhr von Gas in das Innenvolumen (3) aufweist,
**dass** das Innenvolumen (3) die Form einer sich parallel zur Ebene der durchströmten Querschnittsfläche (19) der Sensoröffnung (9) erstreckenden Scheibe, vorzugsweise einer Kreisscheibe, hat,
**dass** die Sensoröffnung (9) und die Membranöffnung (13) einander gegenüberliegend in dem Gehäuse (5) vorgesehen sind und durch das Innenvolumen (3), das als senkrecht zur Flächennormalen (21) auf der durchströmten Querschnittsfläche (19) der Sensoröffnung (9) verlaufende Scheibe ausgebildet ist, in Gasverbindung stehen,
**dass** das Gehäuse (5) einen gegenüber der zentralen Flächennormalen (21) auf der durchströmten Querschnittsfläche (19) der Sensoröffnung (9) konzentrisch um das Innenvolumen (3) ausgebildeten Ringkanal (25) aufweist,
**dass** der Ringkanal (25) in Gasverbindung steht zu dem Innenvolumen (3) und
**dass** der Ringkanal (25) in Gasverbindung steht mit der Gaszuführung (7), und
**dass** der Ringkanal (25) mit dem Innenvolumen (3) über eine Vielzahl von zu der zentralen Flächennormalen (21) auf der durchströmten Querschnittsfläche (19) der Sensoröffnung (9) radial verlaufenden Verbindungskanälen (27) verbunden ist.

2. Sensorkopf nach Anspruch 1, wobei die Abmessung des Innenvolumens (3) in Richtung einer Flächennormalen (21) auf der durchströmten Querschnittsfläche (19) der Sensoröffnung (9) wenigstens um einen Faktor, der größer als 5 ist, kleiner ist als die Abmessungen des Innenvolumens (3) parallel zu dieser Querschnittsfläche (19).

3. Sensorkopf nach einem der Ansprüche 1 bis 2, wobei die Gaszuführung (7) mit einer Gaspumpvorrichtung (23) zur Förderung von Gas in das Innenvolumen (3) verbunden ist.

4. Sensorkopf nach einem der Ansprüche 1 bis 3, wobei auf der von dem Innenvolumen (3) abgewandten Seite der gasdurchlässigen Membran (17) ein die Membran (17) auf dieser Seite vollständig umgebender Schutzgaskanal (33) vorgesehen ist,
wobei der Schutzgaskanal (33) über eine Kanalöffnung (35) mit der umgebenden Atmosphäre (15) in Verbindung steht,
wobei der Schutzgaskanal (33) eine Schutzgaszufuhröffnung (37) aufweist und
wobei die Schutzgaszuführöffnung (37) mit der Gaszuführung (7) verbunden ist.

5. Sensorkopf nach Anspruch 4, wobei die Kanalöffnung (35) der Membranöffnung (13) gegenüber liegt.

6. Sensorkopf nach Anspruch 4 oder 5 wobei die Kanalöffnung (35) durch eine gasdurchlässige Schutzmembran (39) abgedeckt ist.

7. Sensorkopf nach einem der Ansprüche 4-6, wobei auf der von dem Schutzgaskanal (33) abgewandten Seite der Kanalöffnung (35) eine Schutzblende (41) vorgesehen ist, die eine Vielzahl von Durchlassöffnungen (43) zum Durchlass von Gas aus dem Schutzgaskanal (33) zur die Schutzblende (41) umgebende Atmosphäre (15) aufweist.

8. Sensorkopf nach einem der Ansprüche 4 bis 7 wobei auf der von dem Schutzgaskanal (33) abgewandten Seite der Kanalöffnung (35) eine Windschutzeinrichtung (45) vorgesehen ist.

## Claims

1. Sensor head (1) for a gas measuring device comprising
a housing (5) enclosing an interior volume (3),
a sensor device (11) provided on the housing (5) and being in gas flow communication with the interior volume (3), and
a gas permeable membrane (17) provided at the housing and arranged such that gas flowing to the sensor device (11) is flowing through the membrane,
a membrane opening (3) provided in the housing (5), which membrane opening establishes a gas flow communication between the interior volume (3) and the ambient atmosphere surrounding the sensor head (1),
wherein the gas permeable membrane (17) is arranged in front of the membrane opening (13) in such a manner that the membrane completely covers the membrane opening (13),
**characterized in that**
the sensor head comprises a gas feed line (7) provided in the housing for feeding gas into the interior volume (3),
the interior volume (3) has the shape of a disk, preferably of a circular disk, oriented parallel to the plane of the cross-sectional area (19) of the sensor opening (9) through which flow passes,
the sensor opening (9) and the membrane opening (13) are arranged opposite to each other in the housing (5) and are in gas flow communication via the interior volume (3) which is formed as a disk which is oriented perpendicular to the surface normal (21) on the cross-sectional area (19) of the sensor opening (9) through which flow passes,
the housing (5) comprises an annular channel (25) arranged about the interior volume (3) and concentrically with respect to the central surface normal (21) on the cross-sectional surface (19) of the sensor opening (9) through which flow passes,
the annular channel (25) is in gas flow communication with the interior volume (3), and
the annular channel (25) is in gas flow communication with the gas feed line (7), and
the annular channel (25) is connected to the interior volume (3) by a plurality of connecting channels (27) extending radially with respect to the central surface normal (21) of the cross-sectional surface (19) of the sensor opening (9) through which flow passes.

2. Sensor head according to claim 1, wherein the dimension of the interior volume (3) in the direction of the surface normal (21) on the cross-sectional surface (19) of the sensor opening (9) through which flow passes is smaller than the dimensions of the interior volume (3) parallel to this cross-sectional surface (19) by a factor which is larger than 5.

3. Sensor head according to any of the claims 1 to 2, wherein the gas feed line (7) is connected to a gas pump device (23) for feeding gas to the interior volume (3).

4. Sensor head according to any of the claims 1 to 3, wherein on the side of the gas permeable membrane (17) facing away from the interior volume (3) a shield gas channel (33) completely surrounding the membrane (17) on this side is provided,
wherein the shield gas channel (33) is in fluid flow communication with the ambient atmosphere (15) via a channel opening (35),
wherein the shield gas channel (33) comprises a shield gas supply opening (37) and
wherein the shield gas supply opening (37) is connected to the gas feed line (7).

5. Sensor head according to claim 4, wherein the channel opening (35) is located opposite to the membrane opening (13) .

6. Sensor head according to claim 4 or 5, wherein the channel opening (35) is covered by a gas permeable protective membrane (39).

7. Sensor head according to claims 4 - 6, wherein a protective screen (41) is provided on the side of the channel opening (35) facing away from the shield gas channel (33), which protective screen comprises a plurality of passage openings (43) for allowing gas from the shield gas channel (33) to pass to the ambient atmosphere (15) exterior to the protective screen.

8. Sensor head according to any of the claims 4 to 7, wherein a windshield device (45) is provided on the side of the channel opening (35) facing away from the shield gas channel (33).

## Revendications

1. Tête de capteur (1) pour un appareil de mesure de gaz
avec un boîtier (5) qui entoure un volume intérieur (3),
avec un dispositif de capteur (11) prévu sur le boîtier (5), se trouvant en liaison gazeuse avec le volume intérieur (3) par l'intermédiaire d'une ouverture de capteur (9) dans le boîtier (5) et
avec une membrane (17) perméable au gaz prévue sur le boîtier (5) qui est prévue afin d'être traversée par du gaz s'écoulant vers le dispositif de capteur (11),
avec une ouverture de membrane (13) prévue dans le boîtier (5) qui établit une liaison gazeuse entre le volume intérieur (3) et l'atmosphère (15) entourant la tête de capteur (1),
dans lequel la membrane perméable au gaz (17) est agencée avant l'ouverture de membrane (13) de manière à recouvrir complètement l'ouverture de membrane (13),
**caractérisé en ce que**
la tête de capteur présente une alimentation en gaz (7) prévue dans le boîtier (5) pour l'alimentation de gaz dans le volume intérieur (3),
le volume intérieur (3) possède la forme d'un disque s'étendant parallèlement au plan de la surface de section (19) traversée de l'ouverture de capteur (9), de préférence un disque circulaire,
l'ouverture de capteur (9) et l'ouverture de membrane (13) sont prévues à l'opposé l'une de l'autre dans le boîtier (5) et sont en liaison gazeuse par le volume intérieur (3) qui est réalisé comme un disque s'étendant perpendiculairement à la normale de surface (21) sur la surface de section (19) traversée de l'ouverture de capteur (9),
le boîtier (5) présente un canal annulaire (25) réalisé de manière concentrique autour du volume intérieur (3) par rapport à la normale de surface centrale (21) sur la surface de section (19) traversée de l'ouverture de capteur (9),
le canal annulaire (25) est en liaison gazeuse avec le volume intérieur (3) et
le canal annulaire (25) est en liaison gazeuse avec l'alimentation en gaz (7), et
le canal annulaire (25) est relié au volume intérieur (3) par le biais d'une pluralité de canaux de liaison (27) s'étendant radialement par rapport à la normale de surface centrale (21) sur la surface de section (19) traversée de l'ouverture de capteur (9).

2. Tête de capteur selon la revendication 1, dans laquelle la dimension du volume intérieur (3) en direction d'une normale de surface (21) sur la surface de section (19) traversée de l'ouverture de capteur (9) est inférieure au moins d'un facteur qui est supérieur à 5, aux dimensions du volume intérieur (3) parallèlement à cette surface de section (19).

3. Tête de capteur selon l'une quelconque des revendications 1 à 2, dans laquelle l'alimentation en gaz (7) est reliée à un dispositif de pompe à gaz (23) pour le transport de gaz dans le volume intérieur (3).

4. Tête de capteur selon l'une quelconque des revendications 1 à 3, dans laquelle sur le côté éloigné du volume intérieur (3) de la membrane (17) perméable au gaz est prévu un canal de gaz de protection (33) entourant sur ce côté complètement la membrane (17),
dans laquelle le canal de gaz de protection (33) est en liaison par l'intermédiaire d'une ouverture de canal (35) avec l'atmosphère environnante (15),
dans laquelle le canal de gaz de protection (33) présente une ouverture d'alimentation en gaz de protection (37) et
dans laquelle l'ouverture d'alimentation en gaz de protection (37) est reliée à l'alimentation en gaz (7).

5. Tête de capteur selon la revendication 4, dans laquelle l'ouverture de canal (35) se trouve en face de l'ouverture de membrane (13).

6. Tête de capteur selon la revendication 4 ou 5, dans laquelle l'ouverture de canal (35) est recouverte par une membrane de protection (39) perméable au gaz.

7. Tête de capteur selon l'une quelconque des revendications 4 à 6, dans laquelle sur le côté éloigné du canal de gaz de protection (33) de l'ouverture de canal (35) un panneau de protection (41) est prévu, lequel présente une pluralité d'ouvertures de passage (43) pour le passage de gaz du canal de gaz de protection (33) à l'atmosphère (15) entourant le panneau de protection (41) .

8. Tête de capteur selon l'une quelconque des revendications 4 à 7, dans laquelle sur le côté éloigné du canal de gaz de protection (33) de l'ouverture de canal (35) un dispositif de protection contre le vent (45) est prévu.
